# EUROPEAN PATENT APPLICATION

(11) **EP 2 267 152 A1**
(43) Date of publication of application: **29.12.2010**
(21) Application number: 09290498.6
(22) Date of filing: 26.06.2009
(51) Int. Cl.: C12Q 1/48

(54) **Test system for measuring MEST activity as well as methods and uses involving the same**

(71) Applicant: Sanofi-Aventis, 75013 Paris (FR)
(72) Inventor: The designation of the inventor has not yet been filed
(74) Representative: Schneider, Rudolf

(57) **Abstract**

The present invention relates to a test system for measuring MEST activity, a method for screening for a ligand for MEST and the use of the test system for the identification of a MEST ligand, particularly a MEST inhibitor.

## Description

The present invention relates to a test system for measuring MEST activity, a method for screening for a ligand for MEST and the use of the test system for the identification of a MEST ligand, particularly a MEST inhibitor.

Obesity is a medical condition in which excess body fat has accumulated to the extent that it may have an adverse effect on health, leading e.g. to reduced life expectancy. Obesity, defined as increase in fat cell mass and insulin resistance of peripheral tissue (e.g. muscle and liver), associated therewith, is an essential health problem in industrialized countries and is also increasing in developing countries. Obesity and insulin resistance quite often lead to metabolic syndrome and diabetes type II and are, therefore, regarded as causes for these diseases.

Fat cell mass is augmented by increase of the number of fat cells (differentiation) and/or the size of fat cells (deposition of an increased amount of cytoplasmatic lipids per cell). It is suggested that protein "MEST" is involved in the regulation of fat cell size (see, for example, Feitosa et al., 2002; Takahashi et al., 2005; and Nikonova et al., 2008).

The following effects have been observed:
i) mRNA level and protein expression of MEST is dramatically increased in fat tissue of fat-fed and obese animals.
ii) MEST expression is correlated with size of fat cells.
iii) Transgene mice having MEST overexpressed in fat tissue, show increased fat cell-specific gene expression and increased fat cell size (but not number), but reduced muscle mass and total mass of non-fat tissue.
iv) Administration of anti-diabetic drugs ("insulin sensitizer" of the glitazone class) to obese animals reduced expression associated with a reduction of fat cell size and improved insulin sensitivity.
v) Overexpression of MEST in cultured fat cells leads to an increased fat cell differentiation and fat cell-specific gene expression.
vi) MEST mRNA and protein are only detectable in fat tissue of diabetic and overweight humans.
vii) A chromosomal locus at human chromosome 7, which influences the human body mass index (as a criterion for obesity) (7q32.3), is located close to the locus identified for MEST gene, also on chromosome 7 (7q32.2).
viii) Mice having deleted MEST gene (MEST KO mice) show a reduced mass of fat tissue (with normal morphology).
ix) Differences in relative obesity of mice after fat-enriched diet correlate with expression of MEST in epididymal fat tissue, wherein the increased amount of mass is already detectable at the development of obesity and is, therefore, predictive and responsible for pathogenesis of obesity.

Originally, MEST was cloned from a carcinoma cell of mouse (MC12). It is expressed in embryonic and extra-embryonic mesoderm, but usually not in adult tissue. Additionally, MEST was identified in a systematic analysis of imprinted genes by subtraction hybridization of cDNAs of normal and parthenogenetic embryos (only from female genome) as an only paternally expressed gene.

However, the enzymatic or biochemical function of MEST has not been described so far. However, due to its relevance in obesity, it is desirable to identify regulators of MEST, particularly as new therapeutic targets and the treatment of diabetes, metabolic syndrome and/or diabetes type II.

Therefore, it was an object of the present invention to develop a test system for measuring MEST activity, which could be used for the identification of MEST ligands. Surprisingly, it has been found that MEST belongs to the super-family of alpha/beta-fold hydrolases (lipases, esterases, serine proteases and acyl transferases). The overall sequence identity of MEST to glycerol 3-phosphate acyl transferases GPAT1-4 is very low (Lehner and Kuksis, 1996; Lewin et al., 1999; Coleman et al., 2000; Cao et al., 2006). Due to the low sequence identity, MEST could not be identified by hybridization or PCR (using degenerated primers), nor by *in silico* sequence analysis as distantly related GPAT isoform.

However, it could now be shown that MEST has an activity as glycerol 3-phosphate acyl transferase, as shown in the Examples and, based on this finding, test systems have been developed. This finding is of particular relevance as glycerol 3-phosphate acyl transferases are rate-determining in the synthesis of lipids in adipocyts and other peripheral tissue, thereby regulating the size of fat cells. Accordingly, the inhibition of MEST provides an interesting target in therapeutic methods related to obesity and diabetes. This has already been confirmed for the other members of the family of glycerol 3-phosphate acyl transferases (e.g. GPAT 1 and 3) in suitable cell-based assays as well as in animal models (Thuresson, 2004).

Accordingly, a first aspect of the present invention relates to a test system for measuring MEST activity, the test system comprising
i) mesoderm-specific transcript homolog protein (MEST) or a functionally active variant thereof,
ii) an acyl acceptor, such as glycerol-3-phosphate
iii) an acyl donor, such as acyl coenzyme A (CoA), wherein the acyl is a C₁₄ to C₂₂ acyl having 0, 1, 2 or 3 double bonds, and
iv) means for detecting the enzyme activity of MEST transferring the acyl residue from acyl CoA to the acyl acceptor.

The test system of the invention may be used in order to elucidate the function and activity of acyl transferring enzyme MEST. Particularly, the test system may be used to develop, identify and/or characterize agents interacting with MEST, particularly activating or inactivating the same. The identified agents may be interesting therapeutic drugs, which could be used in the treatment of MEST-related diseases, such as obesity and diabetes.

A series of test designs is known in the art to which the test system of the present invention may be adapted. Further details on exemplary tests are given in the methods of the invention. The test system may be used in order to measure the activity of MEST, optionally in the presence of an agent suspected or known to interact with MEST. The skilled person will be able to adapt the test system, e.g. by adding further agents required in connection with the prevailing method, to the particular test design intend.

In accordance with the present invention the test system is designed in order to determine the activity of MEST. MEST is an acyl transferring enzyme catalyzing acylation of a biological molecule. In the present context the acyl transferring enzyme catalyzes acyl transfer from an acyl donor, particularly acyl coenzyme A (CoA) such as palmitoyl-CoA or oleoyl-CoA, to an acyl acceptor, particularly glycerol 3-phosphate.

As detailed above, the test system is used in order to determine the enzyme activity of the acyl transferring enzyme MEST, i.e. its activity in transferring an acyl group from an acyl donor to an acyl acceptor. The enzyme activity is generally defined as the moles of substrate converted per unit time = rate × reaction volume. Enzyme activity is a measure of the quantity of active enzyme present and is thus dependent on conditions. The SI unit is the katal, 1 katal = 1 mol s⁻¹, but this is an excessively large unit. A more practical and commonly-used value is 1 enzyme unit (EU) = 1 µmol min⁻¹ (µ = micro, x 10⁻⁶). 1 U corresponds to 16.67 nanokatals. However, enzyme activity of the acyl transferring enzyme may be also determined as change of the enzyme activity of the acyl transferring enzyme (relative units), e.g. by comparing enzyme activity in the absence and presence of a compound to be tested. An exemplary test design is described in Examples 4 to 6.

Evidently, the enzyme activity in influenced by a series of factors including the amount of enzyme, the activation status of the enzyme, the presence of cofactors such as a co-activator or co-repressor, the presence of activators and inhibitors and the ambient condition such as salt concentration, temperature, pH etc. Usually the enzyme activity is measured at standard laboratory conditions and may be adapted to the optimum of the test system in question. Accordingly, the test system may be used in order to detect or identify molecules changing the activation status of the enzyme, such as activators and inhibitors, which might be useful therapeutics.

As a first component (also referred to as component i)) the test system comprises mesoderm-specific transcript homolog protein (MEST) or a functionally active variant thereof.

Mesoderm-specific transcript homolog protein (MEST) is also referred to as paternally-expressed gene 1 protein (PEG1). Further characteristics of the protein or gene are given in the introductive part of the description.

So far 3 isoforms of MEST have been identified, which are produced by alternative splicing: Isoform 1 (identifier: Q5EB52-1, see Protein knowledgebase UniProtKB at http://www.uniprot.org/), Isoform 2 (identifier: Q5EB52-2), in which amino acids 1-9 of isoform 1 are missing and Isoform 3 (identifier: Q5EB52-3), in which amino acids 1-9 and 218-251 of isoform 1 are missing. Human MEST Isoform 1 has the following amino acid sequence (cf. PRO_0000284418):

10 20 30 40 50 60


70 80 90 100 110 120


130 140 150 160 170 180


190 200 210 220 230 240


250 260 270 280 290 300


310 320 330


(SEQ ID NO: 1)

Isoform 1 is expressed only from the paternal allele, whereas isoform 2 is expressed from both the paternal allele and the maternal allele. Monoallelic expression of the paternally derived allele was observed in all fetal tissues examined, including brain, skeletal muscle, kidney, adrenal, tongue, heart, skin, and placenta.

Due to its catalytic triad (serine 145, histidine 146, aspartate 147) it was suggested that MEST belongs to the AB (fold) hydrolase superfamily (also referred to as α/β hydrolase superfamily). However, its enzymatic or biochemical function was not elucidated before. Known members of AB (fold) hydrolase superfamily are found to be involved in important biochemical processes and related to various diseases. As one of the largest protein superfamilies, AB hydrolase superfamily has gone through an interesting evolutionary process that seemly unrelated amino sequences can conform to structure with similarities. The protein fold of 5 apparently unrelated hydrolases was named as a/b hydrolase fold in the early 1990s. A canonical a/b hydrolase fold consists of an eightstranded parallel a/b structure. Enzymes in this family may have unrelated sequences, various substrates, and different kinds of catalytic activities such as: carboxylic acid ester hydrolase, lipase, thioester hydrolase, peptide hydrolase, haloperoxidase, dehalogenase, epoxide hydrolase and C-C bond breaking enzymes.

Within the present invention it could be shown that MEST has an enzymatic activity as acyl transferase (see also Examples). This finding was particularly surprising as the overall sequence similarity to known acyl transferases, in particular glycerol 3-phosphate acyl transferases (GPAT) 1-4, was quite low. Therefore, MEST has not yet been identified as a remote acyl transferase.

According to the present invention the feature "mesoderm-specific transcript homolog protein" (MEST) relates to any naturally occurring MEST including the human isoforms 1, 2 and 3 as defined above. However, human isoform 1 (cf. SEQ ID NO: 1) as described and illustrated above is particularly preferred.

In addition to any natural occurring MEST isoform or variant, such as a species variants or splice variants, modified MEST proteins may be also used. It should be noted that the modified MEST protein or MEST variant is a functionally active variant, in that the variant maintains its biological function, i.e. its acyl transferring activity. Preferably, maintenance of biological function, e.g. transfer of acyl groups, is defined as having at least 50 %, preferably at least 60 %, more preferably at least 70 %, 80 % or 90 %, still more preferably 95 % of the activity of the naturally occurring MEST. The biological activity may be determined as described in the Examples.

The variant may be a molecule having a domain composed of a naturally occurring MEST protein and at least one further component. For example, the protein may be coupled to a marker, such as a tag used for purification purposes (e.g. 6 His (or HexaHis) tag, Strep tag, HA tag, c-myc tag or glutathione S-transferase (GST) tag). If e.g. a highly purified MEST protein or variant should be required, double or multiple markers (e.g. combinations of the above markers or tags) may be used. In this case the proteins are purified in two or more separate chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a haemagglutinin-epitope-tag), His-MBP (His-tag fused to a maltose-binding protein, FLAG-HA-tag (FLAG-tag fused to a hemagglutinin-epitope-tag), and the FLAG-Strep-tag (FLAG-tag fused to a Strep-tag). The marker could be used in order to detect the tagged protein, wherein specific antibodies could be used. Suitable antibodies include anti-HA (such as 12CA5 or 3F10), anti-6 His, anti-c-myc and anti-GST. Furthermore, the MEST protein could be linked to a marker of a different category, such as a fluorescence marker or a radioactive marker, which allows for the detection of MEST. In a further embodiment, MEST could be part of a fusion protein, wherein the second part could be used for detection, such as a protein component having enzymatic activity.

In another embodiment of the present invention, the MEST variant could be a MEST fragment, wherein the fragment is still capable of transferring acyl groups. This may include MEST proteins with short C- and/or N-terminal deletions (e.g. deletions of at most 20, 19, 18, 17, 16, 15, 14, 13, 12, 11, 10, 9, 8, 7, 6 5, 4, 3, 2, or 1 amino acid). Additionally, the MEST fragment may be further modified as detailed above for the MEST protein.

Alternatively or additionally, the MEST protein or variant thereof as described above may comprise one or more amino acid substitution(s), particularly in regions not involved in the transfer of acyl groups. However, conservative amino acid substitutions, wherein an amino acid is substituted with a chemically related amino acid are preferred. Typical conservative substitutions are among the aliphatic amino acids, amino acids having aliphatic hydroxyl side chains, amino acids having acidic residues, amino acids having amide groups, amino acids having basic residues or amino acids having aromatic residues. The MEST protein or fragment or variant with substitution may be modified as detailed above for the MEST protein or fragment or variant. In the following description of the invention all details given with respect to MEST protein also relate to functionally active variants thereof, unless stated otherwise.

However, most preferably, the MEST protein is a naturally occurring MEST protein, still more preferably, a naturally occurring human MEST protein (isoform 1, 2 or 3) or the functionally active variant T579B (amino acids 2 to 335 of SEQ ID NO: 1) or the functionally active variant T580B (amino acids 11 to 335 of SEQ ID NO: 1). Due to the fact that it has been proven that amino acids 1 to 11 can be omitted it is assumed that any of the following variants would be suitable as well: amino acids 3 to 335 of SEQ ID NO: 1, amino acids 4 to 335 of SEQ ID NO: 1, amino acids 5 to 335 of SEQ ID NO: 1, amino acids 6 to 335 of SEQ ID NO: 1, amino acids 7 to 335 of SEQ ID NO: 1, amino acids 8 to 335 of SEQ ID NO: 1, amino acids 9 to 335 of SEQ ID NO: 1 or amino acids 10 to 335 of SEQ ID NO: 1.

As a second component (also referred to as component ii)) the test system comprises an acyl acceptor. An acyl acceptor is a chemical compound to which the acyl group is donated during the transacylation. In the present invention the acyl transfer is mediated by MEST. Accordingly, any suitable acyl acceptor accepted by MEST may be used. Examples of acyl acceptors typically include phospholipids such as phosphatidic acid, phosphatidylglycerol, phosphatidylserine, phosphatidylcholine, monoacylglycerol, diacylglycerol and, particularly, glycerol-phosphate and dihydroxy acetone phosphate. Preferably, the acyl acceptor is glycerol-phosphate or dihydroxy acetone phosphate, more preferably, glycerol 3-phosphate.

As a third component (also referred to as component iii)) the test system comprises an acyl donor, namely an acyl coenzyme A (CoA), wherein the acyl is a C₁₄ to C₂₂ acyl having 0, 1, 2 or 3 double bonds.

Acyl CoA is a coenzyme involved in the metabolism of fatty acids. It is a temporary compound formed when coenzyme A (CoA) attaches to the end of a long-chain fatty acid, inside living cells. Acyl CoA has the general formula 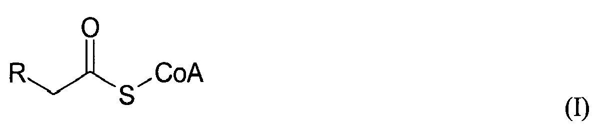 wherein CoA represents coenzyme A and R represents a fatty acid residue having 14 to 22 C atoms.

Coenzyme A (CoA, CoASH, or HSCoA) is a coenzyme, notable for its role in the synthesis and oxidation of fatty acids, and the oxidation of pyruvate in the citric acid cycle. Since coenzyme A is chemically a thiol, it can react with carboxylic acids to form thioesters, thus functioning as an acyl group carrier. It assists in transferring fatty acids. When it is not attached to an acyl group it is usually referred to as 'CoASH' or 'HSCoA'.

Fatty acids are aliphatic monocarboxylic acids derived from, or contained in esterified form in an animal or vegetable fat, oil, or wax. Natural fatty acids commonly have a chain of four to 28 carbons (usually unbranched and even numbered), which may be saturated or unsaturated.

According to the present invention the fatty acids can be saturated (0 double bonds) and unsaturated (1, 2 or 3 double bonds). They differ in length as well and may have from 14 to 22 carbon atoms, particularly 16 to 20 carbon atoms, such as 16, 18 or 20 carbon atoms.

Examples of saturated fatty acids having 14 to 22 carbon atoms include
- myristic acid (tetradecanoic acid CH₃(CH₂)₁₂COOH),
- pentadecylic acid (pentadecanoic acid CH₃(CH₂)₁₃COOH),
- palmitic acid (hexadecanoic acid CH₃(CH₂)₁₄COOH),
- margaric acid (heptadecanoic acid CH₃(CH₂)₁₅COOH),
- stearic acid (octadecanoic acid CH₃(CH₂)₁₆COOH),
- nonadecylic acid (nonadecanoic acid CH₃(CH₂)₁₇COOH),
- arachidic acid (eicosanoic acid CH₃(CH₂)₁₈COOH),
- heneicosylic acid (heneicosanoic acid CH₃(CH₂)₁₉COOH), and
- behenic acid (docosanoic acid CH₃(CH₂)₂₀COOH).

Examples of unsaturated fatty acids having 14 to 22 carbon atoms include
- Myristoleic acid (CH₃(CH₂)₃CH=CH(CH₂)₇COOH),
- Myristoleic acid (CH₃(CH₂)₃CH=CH(CH₂)₇COOH),
- Palmitoleic acid (CH₃(CH₂)₅CH=CH(CH₂)₇COOH),
- Octadeca-6-enoic acid (CH₃(CH₂)₁₀CH=CH(CH₂)₄COOH),
- Oleic acid (CH₃(CH₂)₇CH=CH(CH₂)₇COOH),
- Octadeca-9-enoic acid (CH₃(CH₂)₇CH=CH(CH₂)₇COOH),
- Octadeca-11-enoic acid (CH₃(CH₂)₅CH=CH(CH₂)₉COOH),
- Linoleic acid (CH₃(CH₂)₄CH=CHCH₂CH=CH(CH₂)₇COOH),
- α-Linolenic acid (CH₃CH₂CH=CHCH₂CH=CHCH₂CH=CH(CH₂)₇COOH),
- Octadeca-6,9,12-trienoic acid (CH₃(CH₂)₄CH=CHCH₂CH=CHCH₂CH=CH(CH₂)₄COOH),
- Octadeca-8,10,12-trienoic acid (CH₃(CH₂)₄CH=CHCH=CHCH=CH(CH₂)₆COOH),
- Octadeca-9,11,13-trienoic acid (CH₃(CH₂)3CH=CHCH=CHCH=CH(CH₂)₇COOH),
- Eicosa-9-enoic acid (CH₃(CH₂)₉CH=CH(CH₂)₇COOH),
- Eicosa-11-enoic acid (CH₃(CH₂)₇CH=CH(CH₂)₉COOH),
- Docosa-11-enoic acid (CH₃(CH₂)₉CH=CH(CH₂)₉COOH), and
- Erucic acid (CH₃(CH₂)₇CH=CH(CH₂)₁₁COOH).

As a fourth component (also referred to as component iv)) the test system comprises means for detecting the enzyme activity of MEST transferring the acyl residue from acyl CoA to the acyl acceptor.

Suitable means for detection of the activity of MEST are detailed throughout the present description.

In addition to components i) to iv), the tests system of the invention may comprise one or more further components. Depending from the test design and method of detection the test system may include further components. The skilled person will be capable of adapting the test system to the study design, i.e. be choosing suitable buffers, cofactors or any other necessary agent. Optionally, as a fifth component (also referred to as (test) agent) the test system comprises an agent suspected of altering activity of the acyl transferring enzyme.

The test system may be in a cellular system or a cell-free system, as appropriate under the prevailing conditions.

In a preferred embodiment of the present invention, the acyl CoA is palmitoyl CoA or oleoyl-CoA, preferably palmitoyl CoA.

In another preferred embodiment of the present invention, the acyl acceptor is glycerol 3-phosphate or dihydroxy acetone phosphate, preferably glycerol 3-phosphate.

Accordingly, the following combinations of acyl CoA and acyl acceptor are preferred:
- The acyl CoA is palmitoyl CoA and the acyl acceptor is glycerol 3-phosphate.
- The acyl CoA is palmitoyl CoA and the acyl acceptor is dihydroxy acetone phosphate.
- The acyl CoA is oleoyl-CoA and the acyl acceptor is glycerol 3-phosphate.
- The acyl CoA is oleoyl-CoA and the acyl acceptor is dihydroxy acetone phosphate.

The combination in which the acyl CoA is palmitoyl CoA and the acyl acceptor is glycerol 3-phosphate is most preferred.

In one preferred embodiment a detectable marker is used in order to detect the enzyme activity of MEST transferring the acyl residue from acyl CoA to the acyl acceptor. Accordingly, the acyl acceptor and/or the acyl CoA may be labeled with at least one detectable marker.

A marker (or label) is any kind of substance which is able to indicate the presence of another substance or complex of substances. The marker can be a substance that is linked to or introduced in the substance to be detected. Detectable markers are used in molecular biology and biotechnology to detect e.g. a protein, a product of an enzymatic reaction, a second messenger, DNA etc.

In a preferred embodiment of the present invention the marker is a radiolabel, particularly ³H, ³²P, ³⁵S or ¹⁴C, especially ³H. The marker may be attached to the acyl group to be transferred from the acyl CoA to the acyl acceptor. After transfer occurred labeled acyl CoA and labeled acyl acceptor may be separated based on their different physical or chemical properties and the amount of radioactivity is quantified in order to determine MEST activity. Alternatively, the acyl acceptor may be labeled. After transfer of acyl occurred labeled non-acylated acyl acceptor and labeled acylated acyl acceptor may be separated based on their different physical or chemical properties and the amount of radioactivity quantified in order to determine MEST activity.

In a more preferred embodiment of the present invention palmitoyl CoA is used to be transferred to the radiolabeled acyl acceptor, particularly to radiolabeled glycerol 3-phosphate. A preferred label is ³H.

In another preferred embodiment of the present invention the marker is one or more fluorescence marker(s). Suitable fluorescence markers are described in the context of the methods of the present invention. In general, the details given above concerning radiolabels are also applicable to markers in general and therefore also to fluorescence markers, wherein the radiolabel is replaced with a (fluorescence) marker.

Alternatively, two markers may be used in order to detect proximity of two substances or moieties. The markers may be, e.g. one fluorescent marker and one scintillator (e.g. for a scintillation proximity assay) or two fluorescent markers may be used (e.g. for FRET). In one example the acyl group and the acyl acceptor could be labeled with a first and a second marker. In case the acyl group is transferred from the acyl CoA to the acyl acceptor, and the labels are therefore in close proximity, energy could be transferred from the first to the second label, thus detecting transfer of the acyl group. Examples of suitable marker combinations include
- radiolabels ³H, ³³P, ³⁵S or ¹⁴C, ¹²⁵I combined with scintillator such as Yttrium silicate or polyvinyl-toluene, e.g. compartmented in a microparticle or
- a donor fluorescent marker such as fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives combined with a acceptor fluorescent marker such as LC-Red 610, LC -Red 640, LC-Red 670, LC -Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium).

The test system of the invention as described above may be used in a method for screening for a MEST ligand.

Accordingly, another aspect of the present invention relates to a method for screening for a ligand for MEST, comprising the steps of:
a) contacting the test system of the invention as described above with an agent under conditions conducive to acyl transfer to acyl acceptor,
b) determining the acyl transferring activity of MEST, and
c) detecting an altered acyl transferring activity of MEST relative to a control, thereby identifying the substance as a ligand for MEST.

For the definition of the features of the method of the present invention it is also referred to the definitions and embodiments detailed above in the context of the test system of the present invention.

For the method of the present invention, the test system comprising MEST or a functionally active variant thereof is contacted with an agent. The agent tested with the test system of the present invention may be any test substance or test compound of any chemical nature. It may already be known as a drug or medicament for a disease. Alternatively, it may be a known chemical compound not yet known to have a therapeutic effect in another embodiment and the compound may be a novel or so far unknown chemical compound. The agent may be also a mixture of test substances or test compounds.

In an embodiment of the screening method of the present invention, the test substance is provided in form of a chemical compound library. Chemical compound libraries include a plurality of chemical compounds and have been assembled from any of multiple sources, including chemical synthesized molecules or natural products, or have been generated by combinatorial chemistry techniques. They are especially suitable for high-throughput screening and may be comprised of chemical compounds of a particular structure or compounds of a particular organism, such as a plant. In the context of the present invention, the chemical compound library is preferably a library comprising proteins and polypeptides or small organic molecules. Preferably, a small organic molecule is less than 500 daltons in size, particularly a soluble, non-oligomeric, organic compound.

In the context of the present invention, the test system is contacted with the agent for a time and under conditions suitable for modulating MEST activity and detecting the same. Suitable conditions include appropriate temperature and solution to avoid e.g. denaturation of proteins involved or to maintain viable cells, if present. Suitable conditions will depend on the particular test system chosen and the skilled person will be able to select the same based on its general knowledge. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10° C to 40° C.

After the contacting of the test system with the agent, the effect of the agent on the test system is detected. In the following, a series of different detection systems will be described in more detail. However, it should be understood that these are exemplary and other test systems and methods may be also appropriate.

If the agent has a specific and significant effect on the test system, the agent is identified as modulator of MEST. A modulator of MEST, particularly a ligand, in the context of the present invention means an agent changing, either increasing or decreasing, MEST activity. Preferably, MEST activity is decreased. In the context of the present invention, MEST activity is modified, i.e. increased or preferably decreased, in comparison to a control, if the MEST activity contacted with the modulator is significant lower or higher, respectively, than that of a control (i.e. MEST not contacted with the modulator/ligand). The person skilled in the art knows statistical procedures to assess whether two values are significantly different from each other such as Student's t-test or chi-square tests. Furthermore, the skilled person knows how to select a suitable control.

Controls are a part of the test methods, since they can eliminate or minimize unintended influences (such as background signals). Controlled experiments are used to investigate the effect of a variable on a particular system. In a controlled experiment one set of samples have been (or is believed to be) modified and the other set of samples are either expected to show no change (negative control) or expected to show a definite change (positive control).

In a preferred embodiment, the MEST activity is reduced by at least 10 %, preferably at least 25 %, more preferably at least 50 %, still more preferably at least 75 % and most preferably at least 90 % of the control.

Especially for high-throughput screening, it might be preferable to use a very easy and robust detection system, which comprises as few components as possible. In one embodiment of the present invention, the test system may only comprise components i) to iv) and optionally a potential modulator of MEST activity.

A component of the test system, particularly the acyl group to be transferred, may be labeled in a variety of ways to allow sufficient detection or purification. Common labeling methods may be used for labeling of one or more functional groups of the component. For protein, these could be for example the primary amino groups, present at the N-terminal of each polypeptide chain and the side chain of lysine residues; sulphhydryl groups, present on cysteine residues made available by treating disulphide bonds with reducing agent or by modifying lysine residues with a reagent such as SATA; or carbohydrate groups, usually present in the Fc region of antibodies, which may be oxidized to create active aldehydes for coupling. The component or protein may be labeled with a series of different agents, such as biotin (for avidine-biotin chemistry), enzymes, activated fluorescent dyes for labeling amines, sulphhydryls or other functional groups, e.g. FITC, fluorescein, rhodamine, Cy dyes or Alexa fluos. Radioactive label, such as ³H, ³²P, ³⁵S, ¹²⁵I or ¹⁴C, as well as common enzyme labels, such as penicillinase, horseradish peroxidase and alkaline phosphatase, may be used as well.

For the method of the invention any suitable method of detection may be used. Suitable methods may be chosen depending on the characteristics of the test system and agents to be tested.

For example, interactions of MEST with agents may be measured. A series of tests are known in the art in which the test system may be used and to which the test system may be adapted. This may be a heterogeneous or homogeneous assay. As used herein, a heterogeneous assay is an assay which includes one or more washing steps, whereas in a homogeneous assay such washing steps are not necessary. The reagents and compounds are only mixed and measured.

The test method may be either a continuous assay or a discontinuous assay.

Continuous assays are most convenient, with a single assay giving the rate of reaction with no further work necessary. There are many different types of continuous assays. In spectrophotometric assays, the course of the reaction is followed by measuring a change in absorbance. Fluorescence is when a molecule emits light of one wavelength after absorbing light of a different wavelength. Fluorometric assays use a difference in the fluorescence of substrate from product to measure the enzyme reaction. These assays are in general much more sensitive than spectrophotometric assays, but can suffer from interference caused by impurities and the instability of many fluorescent compounds when exposed to light. Calorimetry is the measurement of the heat released or absorbed by chemical reactions. These assays are very general, since many reactions involve some change in heat and with the use of a microcalorimeter, not much enzyme or substrate is required. These assays can be used to measure reactions that are impossible to assay in any other way. Chemiluminescence is the emission of light by a chemical reaction. Some enzyme reactions produce light and this can be measured to detect product formation. These types of assay can be extremely sensitive, since the light produced can be captured by photographic film over days or weeks, but can be hard to quantify, because not all the light released by a reaction will be detected. Static Light Scattering measures the product of weight-averaged molar mass and concentration of macromolecules in solution. Given a fixed total concentration of one or more species over the measurement time, the scattering signal is a direct measure of the weight-averaged molar mass of the solution, which will vary as complexes form or dissociate. Hence the measurement quantifies the stoichiometry of the complexes as well as the kinetics. Light scattering assays of protein kinetics is a very general technique that does not require an enzyme.

Discontinuous assays are when samples are taken from an enzyme reaction at intervals and the amount of product production or substrate consumption is measured in these samples. Radiometric assays measure the incorporation of radioactivity into substrates or its release from substrates. The radioactive isotopes most frequently used in these assays are ¹⁴C, ³²P, ³⁵S and ¹²⁵I. Since radioactive isotopes can allow the specific labeling of a single atom of a substrate, these assays are both extremely sensitive and specific. They are frequently used in biochemistry and are often the only way of measuring a specific reaction in crude extracts (the complex mixtures of enzymes produced when cells are lysed). Chromatographic assays measure product formation by separating the reaction mixture into its components by chromatography. This is usually done by high-performance liquid chromatography (HPLC), but can also use the simpler technique of thin layer chromatography. Although this approach can need a lot of material, its sensitivity can be increased by labeling the substrates/products with a radioactive or fluorescent tag.

In an embodiment the assay is an SPA (scintillation proximity assay), a FRET (fluorescence resonance energy transfer) assay, TR-FRET (time-resolved fluorescence resonance energy transfer) assay or a FP (fluorescence polarisation) assay.

SPA (scintillation proximity assay) is a type of technology that is used for biochemical screening which permits the rapid and sensitive measurement of a broad range of processes in a homogeneous system. The type of beads that is involved in the SPA are microscopic in size and within the beads itself there is a scintillant which emits light when it is stimulated. Stimulation occurs when radio-labeled molecules interact with the bead. This interaction will trigger the bead to emit light, which can be detected using scintillation counters.

In more detail, when the radio-labeled molecule is attached or is in close proximity to the bead, light emission is stimulated. However, if the bead remains unbounded by the radio-labeled molecule, the bead will not be stimulated to emit light. This is due to the fact that the energy released from the unbounded radioactivity is too diluted when it is too far away from the SPA bead, hence the beads are not stimulated to produce a signal.

Tritium is highly recommended as it suits SPA very well. This is due to the 1.5 µm path length through water, which is very short. So, when the β-particle is within that particular range of 1.5 µm from the scintillant bead, there is sufficient energy to stimulate the scintillant bead to emit light. If the distance is greater than 1.5 µm, then the β-particle is incapable of traveling the required distance to stimulate the bead as there is insufficient energy. There is also an assortment of bead coatings available that allows this method to be applied to a broad range of applications, such as enzyme assays and radio-immuno assays.

Fluorescence resonance energy transfer (FRET) describes a radiation-free energy transfer between two chromophores. A donor chromophore in its excited state can transfer energy by a non-radiative long-range dipole-dipole coupling mechanism to an acceptor fluorophore in close proximity (typically <10 nm). As both molecules are fluorescent, the energy transfer is often referred to as "fluorescence resonance energy transfer", although the energy is not actually transferred by fluorescence. FRET is a useful tool to detect and quantify protein-agent interactions, protein-protein interactions, protein-DNA interactions, and protein conformational changes. For monitoring binding of a protein to an agent, a protein to another protein or a protein to DNA, one of the molecules is labeled with a donor and the other with an acceptor and these fluorophore-labeled molecules are mixed. When they are present in an unbound state, donor emission is detected upon donor excitation. Upon binding of the molecules, the donor and acceptor are brought in proximity and the acceptor emission is predominantly observed because of the intermolecular FRET from the donor to the acceptor. Suitable neighbors for FRET are known in the art and the skilled practitioner will be able to choose a suitable combination of labels for both antibodies. As used herein with respect to donor and corresponding acceptor, "corresponding" refers to an acceptor fluorescent moiety having an emission spectrum that overlaps with the excitation spectrum of the donor. However, both signals should be separable from each other. Accordingly, the wavelength maximum of the emission spectrum of the acceptor should preferably be at least 30 nm, more preferably at least 50 nm, such as at least 80 nm, at least 100 nm or at least 150 nm greater than the wavelength maximum of the excitation spectrum of the donor.

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC-Red 610, LC -Red 640, LC-Red 670, LC -Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (e.g., Europium, or Terbium). Donor and acceptor fluorescent moieties can be obtained, for example, from Molecular Probes (Junction City, OR) or Sigma Chemical Co. (St. Louis, MO).

Alternatively, time-resolved fluorescence resonance energy transfer (TR-FRET) may be used for the test system of the present invention. TR-FRET unites TRF (time-resolved fluorescence) and the FRET principle. This combination combines the low background benefits of TRF and the homogeneous assay format of FRET. While FRET has already been described above, TRF takes advantage of the unique properties of lanthanides or any other donor with long half-life. Suitable donors for TR-FRET include, amongst others, lanthanide chelates (cryptates) and some other metal ligand complexes, which can have fluorescent half-life in the micro- to millisecond time range and which, therefore, also allow the energy transfer to occur in micro- to millisecond measurements. Fluorescence lanthanide chelates have been used as energy donors in the late seventies. The commonly used lanthanides include samarium (Sm), europium (Eu), terbium (Tb) and dysprosium (Dy). Because of their specific photophysical and spectral properties, complexes of lanthanides are of major interest for fluorescence application in biology. Specifically, they have a large stroke's shift and extremely long emission half-lives (from microseconds to milliseconds) when compared to more traditional fluorophores.

Usually, organic chromophores are used as acceptors. These include allophycocyanin (APC). Suitable details on TR-FRET as well as acceptors are described in WO 98/15830.

Fluorescence polarisation (FP)-based assays are assays which use polarized light to excite fluorescent substrate in solution. These fluorescent substrates are free in solution and tumble, causing the emitted light to become depolarised. When the substrate binds to a larger molecule, i.e. the acyl group, its tumbling rates are greatly decreased, and the emitted light remains highly polarized.

Alternatively, mass spectrometry may be used. The term "mass spectrometry" refers to the use of an ionization source to generate gas phase ions from a sample on a surface and detecting the gas phase ions with a mass spectrometer. The term "laser desorption mass spectrometry" refers to the use of a laser as an ionization source to generate gas phase ions from a sample on a surface and detecting the gas phase ions with a mass spectrometer. A preferred method of mass spectrometry for biomolecules such as acylated acyl acceptor is matrix-assisted laser desorption/ionization mass spectrometry or MALDI. In MALDI, the analyte is typically mixed with a matrix material that, upon drying, co-crystallizes with the analyte. The matrix material absorbs energy from the energy source which otherwise would fragment the labile biomolecules or analytes. Another preferred method is surface-enhanced laser desorption/ionization mass spectrometry or SELDI. In SELDI, the surface on which the analyte is applied plays an active role in the analyte capture and/or desorption. In the context of the invention the sample comprises a biological sample that may have undergone chromatographic or other chemical processing and a suitable matrix substrate.

In mass spectrometry the "apparent molecular mass" refers to the molecular mass (in Daltons)-to-charge value, m/z, of the detected ions. How the apparent molecular mass is derived is dependent upon the type of mass spectrometer used. With a time-of-flight mass spectrometer, the apparent molecular mass is a function of the time from ionization to detection. The term "signal" refers to any response generated by a biomolecule under investigation. For example, the term signal refers to the response generated by a biomolecule hitting the detector of a mass spectrometer. The signal intensity correlates with the amount or concentration of the biomolecule. The signal is defined by two values: an apparent molecular mass value and an intensity value generated as described. The mass value is an elemental characteristic of the biomolecule, whereas the intensity value accords to a certain amount or concentration of the biomolecule with the corresponding apparent molecular mass value. Thus, the "signal" always refers to the properties of the biomolecule.

In a preferred embodiment of the invention at least one of the starting materials acyl acceptor and the acyl CoA is labeled with at least one detectable marker and wherein after step a) and before step b) the labeled starting material is separated from the labeled product acylated acyl acceptor. The separation may be done by a common separation step such as centrifugation, immobilization and removal of liquids etc. Alternatively, it may be done by using different solvents and phase separation, e.g. as described in the Examples. In a preferred embodiment the separating is by use of an organic and an aqueous phase, wherein the acylated acyl acceptor accumulates in the organic phase and the non-acylated acyl acceptor accumulates in the aqueous phase.

In another preferred embodiment of the methods of the present invention the activity of MEST is determined by detecting radioactivity. Suitable markers and methods are detailed herein.

In a preferred embodiment of the methods of the present invention the activity of MEST is determined by detecting fluorescence. Suitable markers and methods are detailed herein.

In a preferred embodiment of the present invention the acyl transferring activity of MEST is by determining the amount of acyl glycerol 3-phosphate, particularly of labeled acyl glycerol 3-phosphate.

The test may be a heterogeneous or homogeneous assay. Preferably, the method is a homogeneous assay. As used herein, a heterogeneous assay is an assay which includes one or more washing steps, whereas in a homogeneous assay such washing steps are not necessary. The reagents and compounds are only mixed and measured.

In a preferred embodiment of the present invention the acyl transferring activity of MEST is decreased relative to a control, thereby identifying the substance as a MEST inhibitor.

However, in an even more preferred embodiment of the present invention, the method is essentially carried out as detailed in the Examples.

This method is based on the MEST-mediated incorporation of acyl with acyl CoA as acyl donor into a radio-labeled acyl acceptor, such as radio-labeled glycerol 3-phosphate, particularly [³H] glycerol 3-phosphate. After MEST has reacted, the labeled product (i.e. acylated labeled acyl acceptor) is separated from non-reacted labeled acyl acceptor by addition of an organic solvent. Due to phase separation, hydrophobic and amphiphilic substances are accumulated in the organic solvent, whereas the hydrophilic substances remain in the aqueous phase. In accordance with the above-detailed method, acylated labeled acyl acceptor is accumulated in the organic phase and non-reacted labeled acyl acceptor is accumulated in the aqueous phase, in which the reaction of MEST has been carried out. The organic solvent may encompass a scintillator allowing for detecting the amount of product in a scintillation counter. In a very preferred embodiment, MEST activity is determined by using palmitoyl CoA acyl donor and glycerol 3-phosphate as acyl acceptor, wherein palmitate is transferred from the acyl donor to the acyl acceptor in the presence of MEST, and wherein glycerol 3-phosphate is labeled, particularly labeled with ³H. The described assay is a homogeneous assay and can be carried out in an easy and fast manner, and allows for high-throughput screening. It is based on a known method used for determining the activity of glycerol 3-phosphate acyl transferases GPAT1-4 (Haldar and Vancura, 1992; Yet et al., 1995, Cao et al., 2000, and Wendel et al., 2008).

Preferably, the method is adapted for high-through put screening. In this method a large number of compounds is screened against the agents in either cell-free or whole-cell assays. Typically, these screenings are carried out in 96 well plates using automated, robotic station based technologies or in higher- density array ("chip") formats.

The test system of the invention may comprise a cell, particularly a mammalian cell, especially a human cell or an insect cell. Examples of suitable cells include Sf9 cells (see Examples). The cells may be e.g. primary cells or a cell line. However, any other cell or cell line, optionally genetically modified to include MEST (see Examples) or components needed for detection of an effect, may be used. In a preferred embodiment cell membranes (crude, fractionated or purified) may be used. Exemplary methods for producing cell membranes from intact cells are detailed in the Examples.

In a preferred method of the present invention the effect is determined by fluorescence. Suitable methods are detailed above and may involve a fluorescence marker, FRET, fluorescence polarization, as detailed herein.

In another preferred embodiment of the invention, the method is used to screen for a medicament for preventing and/or treating a disease involving MEST dysfunction. Particularly, the method is used for screening for a medicament for preventing and/or treating obesity and/or diabetes, such as diabetes type II.

In accordance with the present invention the term "prevention of a disease" relates to the reduction of the risk of developing the prevailing disease, whereas the term "treatment of a disease" relates to the amelioration of the symptoms of the prevailing disease condition, deceleration of the course of disease etc. A prevention or preventive measure is a way to avoid an injury, sickness, or disease in the first place. A treatment or cure is applied after a medical problem has already started. A treatment treats a health problem, and may lead to its cure, but treatments more often ameliorate a problem only for as long as the treatment is continued. Cures are a subset of treatments that reverse illnesses completely or end medical problems permanently.

A further aspect of the present invention relates to the use of a test system according to the invention for the identification of a MEST ligand, particularly a MEST inhibitor.

A ligand is a substance that is able to bind to and form a complex with a biomolecule, herein MEST. It is molecule binding to a site on MEST, by intermolecular forces such as ionic bonds, hydrogen bonds, hydrophobic interactions and Van der Waals forces. The docking (association) is usually reversible (dissociation). Actually, irreversible covalent binding between a ligand and its target molecule is rare in biological systems. Ligand binding to an enzyme such as MEST may alter the enzymatic activity of the enzyme. Ligands include inhibitors and activators.

Enzyme inhibitors are molecules that bind to enzymes and decrease their activity. Since blocking an enzyme's activity can correct a metabolic imbalance, many drugs are enzyme inhibitors. Not all molecules that bind to enzymes are inhibitors; enzyme activators bind to enzymes and increase their enzymatic activity.

The binding of an inhibitor can stop a substrate from entering the enzyme's active site and/or hinder the enzyme from catalyzing its reaction. Inhibitor binding is either reversible or irreversible. Irreversible inhibitors usually react with the enzyme and change it chemically. These inhibitors modify key amino acid residues needed for enzymatic activity. In contrast, reversible inhibitors bind non-covalently and different types of inhibition are produced depending on whether these inhibitors bind the enzyme, the enzyme-substrate complex, or both.

Selective ligands have a tendency to bind to very limited types of targets, such as enzymes, while non-selective ligands bind to several types of targets. This plays an important role in pharmacology, where drugs that are non-selective tend to have more adverse effects, because they bind to several other targets, such as enzymes, in addition to the one generating the desired effect.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods and materials are described herein.

The invention is further illustrated by the following examples, although it will be understood that the examples are included merely for purposes of illustration and are not intended to limit the scope of the invention unless otherwise specifically indicated.

### EXAMPLES

### METHOD

The assay for determining glycerol 3-phosphate acyl transferase is based on the following principle:

Glycerol 3-phosphate acyl transferase activity was determined as incorporation of palmitate with palmitoyl CoA as acyl donor and radio-labeled [³H] glycerol 3-phosphate as acyl acceptor, to obtain radio-labeled palmitoyl glycerol 3-phosphate. Radioactive-labeled product palmitoyl glycerol 3-phosphate was separated from the radio-labeled substrate by addition of a scintillator based on organic solvent in order to determine radioactivity in a suitable counter. After separation of phases, only radio-label in the organic solvent phase, which is saturated with scintillator, is detected, i.e. hydrophobic and amphiphilic substances as radio-labeled palmitoyl glycerol 3-phosphate. Hydrophilic radio-labeled glycerol phosphate, which has not been acylated, remains in the aqueous phase and is, therefore, not counted by the scintillation counter. The low amount of "cross-irradiation" from the aqueous phase to the organic phase was corrected by substracting a blank value of a control (containing all reaction components apart from MEST protein) for all MEST reactions.

The following solutions were used for the assay:

### A) Reaction solution:

95 mM Tris/HCl pH=7.4 (MERCK, # 1.08382.2500),
5 mM MgCl₂ (SIGMA, # 104-20)
2.5 mg/mL BSA (SIGMA, # A3803)
125 µM Palmitoyl CoA (SIGMA, # P9716)
187.5 µM glycerol 3-phosphate (SIGMA, # G7886)
150 nCi/well ³H glycerol 3-phosphate (ARC, #ART0219)

### B) Homogenization buffer:

TES buffer (20 mM Tris/HCl pH=7.4, 1 mM EDTA, 250 mM sucrose)
+ 10 µg/mL Leupeptin (BIOMOL, #12136)
+ 10 µg/mL Aprotinine (USB Corporation, #11388)
+ 10 µg/mL Pepstatin A (BIOMOL, #17640)
+ 100 mM Pefabloc (MERCK, #1.24839.0500)

### C) Enzyme solution:

Membranes of insect cells expressing human recombinant MEST protein (as below) in homogenization buffer adjusted to the desired concentration (determination of protein content by BioRad assay)

### Format:

96-well plate (wallac isoplate #6005040)

### Assay design

10 µl enzyme solution were incubated with 50 µl reaction solution at 25 °C for 60 min. Incubation was carried out in an Eppendorf Thermomixer comfort with gentle agitation (450 min⁻¹), wherein the openings of the plate were sealed by a protection film (Whatman Uni Seal 7704-0009). The reaction was terminated by addition of 5 µl 200 mM glycerol 3-phosphate and 200 µl scintillation liquid (beta-plate, # 1205-440, Perkin Elmer) and incubation under agitation as above at 25 °C for 15 min. After at least 12 hours of incubation at 25 °C without agitation for separation of the phases, radioactivity (in dpm) was measured in a scintillation counter (Wallac 1450 micro beta).

The enzyme solution (total membranes of Sf9 insect cells) was produced as follows:
Sf9 insect cells were pelleted by centrifugation and frozen in aliquots (about 7-9 g). When needed, pelleted Sf9 insect cells were quickly thawed in a 37 °C water bath and kept on ice until further processing. Thereafter, they were transferred into a 50 ml glass homogenizer containing 10 ml homogenization buffer and, additionally, 0.5 mM DTT for preparing a suspension. Cells were homogenized in a teflon-in-glass homogenizer (10 x 1500/min at 4 °C). The homogenate was transferred into JA25.5 centrifugation tubes (Beckman #363647), diluted with 10 ml homogenization buffer plus DTT and pelleted by centrifugation (500 g, 5 min, 4 °C, refrigerated centrifuge Beckman Avanti JA251). The supernatant was transferred into ultracentrifugation tubes (Beckman #355618) and centrifugated (100000 g, 60 min, 4 °C, Beckman Optima LE-80K). The supernatant was completely removed. The pellet was two times carefully washed with homogenization buffer and then resuspended in homogenization buffer. Enzyme solution was aliquoted, frozen in liquid nitrogen and stored at -80 °C.

The construction of MEST coding transfer vectors was carried out as follows:
The mRNAs coding for MEST proteins (T579B = amino acids 2-335 and T580B = amino acids 11-335) (two different start codons, cf. Sado et al., 1993; Kaneko-Ishino et al., 1995) were cloned by PCR using hypothalamic cDNA as template. One of the PCR primers (5'-GAGAG AATTC GATGG TGCGC CGAGA TCGCT T-3' SEQ ID NO: 2) comprises a EcoRI restriction site having in frame a ATG-start codon at the 5' terminus of the MEST-cDNA. The second PCR primer (5'-GAGAG CGGCC GCTCA GAAGG AGTTG ATGAA GC-3', SEQ ID NO: 3) comprises a TGA stop codon and a Not1 restriction site at the 3' terminus. The amplified DNA was cloned inti the EcoRI and NotI restriction sites of the insect cell expression vector pVL1392 (PharMingen). Identity of the cloned gene was confirmed by direct DNA sequencing.

Sf9 insect cells were produced and propagated as follows:
All methods including Sf9 cell culture, sub-cloning, infections, etc. were carried out in accordance with the method known from literature (see, for example, Summers and Smitz, 1987). Typically, 3 x 10⁶ Sf9 cells (PharMingen) were seeded in a T25 bottle in serum-free medium (Sf-900 II SFM, GIBCO/BRL). After adherence of the cells, the medium was replaced with TMN-FH insect medium (PharMingen). The recombinant transfection vector was co-transfected with inactivated linearized BaculoGold DNA (PharMingen) in Sf9 insect cells, in order to produce a recombinant Baculo virus with a recombination efficiency of almost 100 %. The resulting recombinant virus stock was further amplified in Sf9 cells for recombinant protein expression. Cells were typically harvested after 3 days.

### Example 1: Glycerol 3-Phosphate Acyl Transferase Activity of MEST - Dependence on the Amount

The example was carried out as detailed above. Particularly, insect cells (SF9) non-transfected or transfected to express either MEST (T579B or T580B) or GPAT1 were produced. Crude membranes of these cells were obtained and used to detect acyl transferring activity (60 min incubation time). As shown in table 1, insect cells transfected with MEST (T579B or T580B) showed significant acyl transferring activity (in dpm per test), even exceeding that of GPAT1. Additionally, it could be shown that activity increased with the amount of protein.

**Table 1: Glycerol 3-Phosphate Acyl Transferase Activity of MEST (dpm) - Comparison of MEST-recombinant Sf9 crude membranes, non-transfected Sf9 crude membranes and GPAT1-recombinant Sf9 crude membranes**

| **Enzyme solution** | **Protein [µg/Test]** | | | | |
|---|---|---|---|---|---|
| | **100** | **30** | **10** | **3** | **1** |
| **SF9** (non-transfected) | 164 | 192 | 100 | 12 | 12 |
| **MEST T579B** (recombinant SF9) | **983** | **1061** | **812** | **312** | **99** |
| **MEST T580B** (recombinant SF9) | **1080** | **1080** | **583** | **340** | **31** |
| **GPAT1** (control) | 584 | 968 | 1702 | 845 | 278 |

### Example 2: Glycerol 3- Phosphate Acyl Transferase Activity of MEST - Time Course

The example was carried out as detailed above. Particularly, insect cells (SF9) transfected to express MEST (T579B or T580B) were produced. Crude membranes of these cells were obtained and used to detect acyl transferring activity (in dpm per test). As shown in table 2, activity increased with time (and the amount of the crude membranes).

**Table 2: Glycerol 3- Phosphate Acyl Transferase Activity of MEST (dpm) - Comparison of different amounts of MEST (T579B, T580B)- recombinant Sf9 crude membranes**

| **Enzyme solution** | **Time [min]** | | | | | | | | | | |
|---|---|---|---|---|---|---|---|---|---|---|---|
| | **0** | **2** | **5** | **10** | **15** | **20** | **30** | **45** | **60** | **90** | **120** |
| **T579B** 30 µg/Test | -16 | 8 | **62** | **166** | **283** | **350** | **560** | **905** | **1041** | **1164** | **1235** |
| **T579B** 10 µg/Test | 3 | 24 | **30** | **107** | **151** | **229** | **404** | **579** | **890** | **1286** | **1670** |
| **T580B** 30 µg/Test | -2 | 20 | **82** | **148** | **224** | **372** | **546** | **947** | **901** | **993** | **1142** |
| **T580B** 10 µg/Test | -3 | 18 | **34** | **68** | **160** | **211** | **350** | **537** | **704** | **1100** | **1669** |

### Example 3: Glycerol 3-Phosphate Acyl Transferase Activity of MEST: Resistance toward NaCl Extraction

The example was carried out as detailed above. Particularly, insect cells (SF9) non-transfected or transfected to express either MEST (T579B or T580B) or GPAT (GPAT1, -3 or -4) were produced. Crude membranes were prepared (see above) and incubated with 1 M NaCl for 1 h at 4°C. After centrifugation (100,000 x *g*, 60 min, 4°C), pellet and supernatant fractions were used to detect acyl transferring activity. As shown in table 3, MEST (T579B or T580B) activity (in dpm/test) was predominately associated with the pelleted membrane rather than supernatant fractions. This indicates that MEST activity is due to an integral rather than peripheral membrane protein.

**Table 3: NaCl-resistant Association of Glycerol 3- Phosphate Acyl Transferase Activity of MEST (dpm)-Comparison of Sf9 crude membranes recombinant for MEST, GPAT1, GPAT3 and GPAT4**

| **Pellets** (obtained by centrifugation 100,000 x *g*, 60 min, 4°C) | | | | | | | |
|---|---|---|---|---|---|---|---|
| | | **Protein [µg/Test]** | | | | | |
| | | **245** | **122.5** | **49** | **24.5** | **12.25** | **4,9** |
| **MEST T579B** | 1M | | | | | | |
| | NaCl | **410** | **701** | **869** | **625** | **532** | **148** |
| | Control | **495** | **739** | **1116** | **1211** | **541** | **280** |
| | | | | | | | |

| | | **Protein [µg/Test]** | | | | | |
|---|---|---|---|---|---|---|---|
| | | **425** | **212.5** | **85** | **42.5** | **21.25** | **8.5** |
| **GPAT1** | 1M | | | | | | |
| | NaCl | 494 | 454 | 1622 | 3134 | 2810 | 1727 |
| | Control | 279 | 541 | 1257 | 2026 | 2060 | 2243 |
| | | | | | | | |

| | | **Protein [µg/Test]** | | | | | |
|---|---|---|---|---|---|---|---|
| | | **84** | **42** | **16.8** | | | |
| **GPAT3** | 1M | | | | | | |
| | NaCl | 817 | 962 | 647 | | | |
| | Control | 552 | 868 | 543 | | | |
| | | | | | | | |

| | | **Protein [µg/Test]** | | | | | |
|---|---|---|---|---|---|---|---|
| | | **72** | **36** | **14.4** | | | |
| **GPAT4** | 1M | | | | | | |
| | NaCl | 710 | 757 | 726 | | | |
| | Control | 562 | 890 | 652 | | | |
| | | | | | | | |
| | | | | | | | |

| **Supernatants** (obtained by centrifugation 100,000 x *g*, 60 min, 4°C) | | | | | | | |
|---|---|---|---|---|---|---|---|
| | | **Supernatant [µL/Test]** | | | | | |
| | | **10** | **5** | **2** | | | |
| **MEST T579B** | 1M | | | | | | |
| | NaCl | **-28** | **-2** | **1** | | | |
| | Control | **1** | **-13** | **-17** | | | |
| | | | | | | | |

| | | **Supernatant [µL/Test]** | | | | | |
|---|---|---|---|---|---|---|---|
| | | **10** | **5** | **2** | | | |
| **GPAT1** | 1M | | | | | | |
| | NaCl | 644 | 369 | 121 | | | |
| | Control | 816 | 410 | 124 | | | |
| | | | | | | | |

| | | **Supernatant [µL/Test]** | | | | | |
|---|---|---|---|---|---|---|---|
| | | **10** | **5** | **2** | | | |
| **GPAT3** | 1M | | | | | | |
| | NaCl | 7 | 6 | -13 | | | |
| | Control | -21 | -3 | -1 | | | |
| | | | | | | | |

| | | **Supernatant [µL/Test]** | | | | | |
|---|---|---|---|---|---|---|---|
| | | **10** | **5** | **2** | | | |
| **GPAT4 (UZ4)** | 1M | | | | | | |
| | NaCl | 13 | 43 | -5 | | | |
| | control | 4 | -2 | -3 | | | |
| | | | | | | | |
| "Blank value" = 163 | | | | | | | |

### Example 4: Partial Inhibition of MEST by Epoxide hydrolase inhibitor A003564556, but not by general Lipase inhibitor S987600

The experiment was carried out as detailed above. Particularly, crude membranes from insect cells (SF9) transfected to express MEST (T579B) were produced and assayed for glycerol 3-phosphate acyltransferase activity (dpm/test) in the presence of A003564556 or S987600. As shown in table 4, MEST activity was partially inhibited by the epoxide hydrolase inhibitor, A003564556 (=AUDA; Dorrance et al. 2005; Kim et al. 2007; Carroll et al. 2008), but not by general Lipase inhibitor S987600 (Petry et al. 2004; Ben Ali et al. 2004 and 2006).

**Table 4: Partial Inhibition of Glycerol 3- Phosphate Acyl Transferase Activity of MEST by Epoxide hydrolase inhibitor A003564556, but not by general Lipase inhibitor S987600**

| **MEST T579B** | | | | |
|---|---|---|---|---|
| (10 µg/Test) | **Activity (dpmlTest)** | | **% Inhibition** | |
| **Concentration in µM** | **A003564556** | **S987600** | **A003564556** | **S987600** |
| 200 | 458 | 995 | **48** | -16 |
| 100 | 557 | 1015 | **34** | -20 |
| 30 | 779 | 1099 | **8** | -29 |
| 10 | 845 | 1139 | **1** | -34 |
| 3 | 881 | 997 | -4 | -17 |
| 1 | 1011 | 1112 | -19 | -31 |
| 0.3 | 905 | 1027 | -6 | -21 |

### Example 5: Inhibition of MEST by the competitive substrate, analog dihydroxy acetone phosphate (DHAP)

The experiment was carried out as detailed above. Particularly, crude membranes of insect cells (SF9) transfected to express either MEST (T579B) or GPAT (GPAT1, -3 or -4) were produced and assayed in the presence of competitive substrate analog dihydroxy acetone phosphate (DHAP). As shown in table 5, MEST activity was inhibited by DHAP in concentration-dependent fashion.

**Table 5: Inhibition of Glycerol 3- Phosphate Acyl Transferase Activity of MEST by competitive substrate analog dihydroxy acetone phosphate (DHAP) - Comparison of Sf9 membranes recombinant for MEST, GPAT1, GPAT3 and GPAT4**

| **Activity (dpm/Test)** | | | | | |
|---|---|---|---|---|---|
| **DHAP** [mM] | **GPAT1** | **GPAT3** | **GPAT4** | **MEST T579B** | **MEST T580B** |
| **10** | 495 | 433 | 597 | 311 | 241 |
| **3** | 950 | 621 | 909 | 502 | 556 |
| **1** | 1266 | 741 | 1170 | 738 | 831 |
| **0.3** | 1593 | 883 | 1089 | 776 | 934 |
| **0.1** | 1597 | 1034 | 1313 | 1076 | 918 |
| **0.03** | 1707 | 1084 | 1216 | 1070 | 1082 |
| **none** | 1780 | 999 | 1288 | 1017 | 1017 |

| **% Inhibition** | | | | | |
|---|---|---|---|---|---|
| **DHAP** [mM] | **GPAT1** | **GPAT3** | **GPAT4** | **MEST T579B** | **MEST T580B** |
| **10** | 72 | 57 | 54 | **69** | **76** |
| **3** | 47 | 38 | 29 | **51** | **45** |
| **1** | 29 | 26 | 9 | **27** | **18** |
| **0.3** | 11 | 12 | 15 | **24** | **8** |
| **0.1** | 10 | -4 | -2 | -6 | **10** |
| **0.03** | 4 | -9 | 6 | -5 | -6 |

### Example 6: Complete/Partial Inhibition of the Glycerol 3-Phosphate Acyltransferase Activity of MEST by N-Ethylmaleimide (NEM) or High Temperature

The experiment was carried out as detailed above. Particularly, crude membranes of non-transfected insect cells (SF9) or insect cells (SF9) transfected to express either MEST (T579B) or GPAT (GPAT1, -3 or -4) were produced and assayed in the presence of 1 mM N-Ethylmaleimide (NEM) for 1 h or at 41°C for 10 min. As shown in table 6, MEST activity (and endogenous insect cell glycerol 3-phosphate acyltransferase activities) was inhibited by NEM (completely) and by high temperature (partially).

**Table 6: Complete/Partial Inhibition of Glycerol 3- Phosphate Acyl Transferase Activity of MEST by 1 mM N-Ethylmaleimide (NEM) or Incubation at 41 °C - Comparison of Sf9 crude membranes recombinant for MEST and endogenous non-transfected Sf9 crude membranes**

| | **30 µg Protein/Test** | | | **10 µg Protein/Test** | | |
|---|---|---|---|---|---|---|
| | **SF9*** | **MEST T579B** | **MEST T580B** | **SF9*** | **MEST T579B** | **MEST T580B** |
| **Activity (dpm/Test)** | | | | | | |
| Control | 165 | 878 | 772 | 120 | 505 | 731 |
| 10'41°C | -2 | 342 | 201 | -18 | 136 | 90 |
| 1 mM NEM | -12 | 53 | 31 | 6 | 49 | -11 |

| **% Inhibition** | | | | | | |
|---|---|---|---|---|---|---|
| | **30 µg Protein/Test** | | | **10 µg Protein/Test** | | |
| | **SF9*** | **MEST T579B** | **MEST T580B** | **SF9*** | **MEST T579B** | **MEST T580B** |
| 10'41°C | 101 | **61** | **74** | 115 | **73** | **88** |
| 1 mM NEM | 107 | **94** | **96** | 95 | **90** | **101** |

| | | | | | | |
|---|---|---|---|---|---|---|
| * non-transfected | | | | | | |

### SUMMARY OF THE RESULTS

Human MEST protein recombinantly produced (two variants with different start codons, differing in the first 11 amino acids) were tested for potential glycerol 3-phosphate acyl transferase activity in a suitable test system (using a radio-label and validated by known acyl transferases). In the test system provided, both variants showed glycerol 3-phosphate acyl transferase activity which was concentration- and time-dependent. The enzyme seems to be bound to crude membranes derived from Sf9 cells after recombinant expression of the protein. Furthermore, glycerol 3-phosphate acyl transferase activity is sensitive to temperature as well as to the irreversible inhibitor NEM and a competitive substrate analogue DHAP. Partial inhibition could be obtained using a reversible inhibitor for members of the epoxide hydrolase family, A003564556 (IC₅₀ ∼ 200 µM).

### REFERENCES

- Ben Ali Y., Chahinian H., Petry S., Müller G., Carriere F., Verger R., Abousalham A. (2004) Biochemistry 43, 9298-9306.
- Ben Ali Y., Chahinian H., Petry S., Müller G., Lebrun R., Verger R., Carriere F., Mandrich L., Rossi M., Manco G., Sarda L., Abousalham A. (2006) Biochemistry 45, 14183-14191, 2006
- Cao J., Li J.-L., Li D., Tobin J.F., Gimeno R.E: (2006) Proc. Natl. Acad. Sci. USA 103, 19695-19700.
- Carroll L.J., Chander P.N., Falck J.R., Sangras B., Stier C.T. (2008) Front Biosci. 13, 3480-3487.
- Coleman R.A., Lewin T.M., Muoio D.M. (2000) Annu. Rev. Nutr. 20, 77-103.
- Dorrance A.M., Rupp N., Pollock D.M., Newman J.W., Hammock B.D., Imig J.D. (2005) J. Cardiovasc. Pharmacol. 46, 842-848.
- Feitosa M., Borecki I., Rich S, Arnett D, Sholinsky P., Myers R., Leppert M., Province M. (2002) Am. J. Hum. Genet. 70, 72-82.
- Haldar D., Vancura A. (1992) Methods Enzymol. 209, 64-72.
- Kaneko-Ishino T., Kuroiwa Y., Miyoshi N., Kohda T., Suzuki R., Yokoyama M., Viville S., Barton S., Ishino F., Surani M. (1995) Nat. Genet. 11, 52-59.
- Kim I.H., Nishi K., Tsai H.J., Bradford T., Koda Y., Watanabe T., Morisseau C., Blanchfield J., Toth I., Hammock B.D. (2007) Bioorg. Med. Chem. 15, 312-323.
- Lehner R., Kuksis A. (1996) Prog. Lipid Res. 35, 169-201.
- Lewin T.M., Wang P., Coleman R.A. (1999) Biochemistry 38, 5764-5771.
- Nikonova L., Koza R.A., Mendoza T., Chao P.-M., Curley J.P., Kozak L.P. (2008) FASEB J. 22, 3925-3937
- Petry S., Baringhaus K.-H., Schönafinger K., Jung C., Kleine H., Müller G. (2004) in Lipases and Phospholipases in Drug Development - From Biochemistry to Molecular Pharmacology, Müller G. and Petry S. (ed.) pp 121-137, Wiley-VCH Weinheim/Germany
- Sado T., Nakajima N., Tada T., Takagi N. (1993) Dev. Growth Differ. 35, 551-560.
- Summers M.D., Smith G.E. (1987) Tex. Agric. Exp. Stn. (Bull.) 1555, 1-57.
- Takahashi M., Kamei Y., Ezaki O. (2005) Am. J. Physiol. Endocrinol. Metab. 288, E117-E124.
- Thuresson E.R. (2004) Curr. Opin. Invest. Drugs 5, 411-418.
- Wendel A.A., Lewin T.M., Coleman R.A. (2008) Biochim. Biophys. Acta doi:10.1016/j.bbalip.2008.10.010.
- Yet S.-F., Moon Y.K., Sul H.S. (1995) Biochemistry 34, 7303-7310.

## Claims

1. A test system for measuring MEST activity, the test system comprising
i) mesoderm-specific transcript homolog protein (MEST) or a functionally active variant thereof,
ii) an acyl acceptor,
iii) an acyl coenzyme A (CoA), wherein the acyl is a C₁₄ to C₂₂ acyl having 0, 1, 2 or 3 double bonds, and
iv) means for detecting the enzyme activity of MEST transferring the acyl residue from acyl CoA to the acyl acceptor.

2. The test system of claim 1,
a) wherein the acyl CoA is palmitoyl CoA or oleoyl-CoA, preferably palmitoyl CoA; and/or
b) wherein the acyl acceptor is glycerol 3-phosphate or dihydroxy acetone phosphate, preferably glycerol 3-phosphate.

3. The test system of claim 1 or 2, wherein the acyl acceptor and/or the acyl CoA is labeled with at least one detectable marker.

4. The test system of claim 1 to 3, wherein the marker is a radiolabel, particularly ³H, ³²P, ³⁵S or ¹⁴C, especially ³H.

5. The test system of any of claims 1 to 4, wherein the marker is one or more fluorescence marker(s).

6. A method for screening for a ligand for MEST, comprising the steps of:
a) contacting the test system according to any of claims 1 to 5 with an agent under conditions conducive to acyl transfer to glycerol 3-phosphate,
b) determining the acyl transferring activity of MEST, and
c) detecting an altered acyl transferring activity of MEST relative to a control, thereby identifying the substance as a ligand for MEST.

7. The method of claim 6, wherein at least one of the starting materials acyl acceptor and acyl CoA is labeled with at least one detectable marker and wherein after step a) and before step b) the labeled starting material is separated from the labeled product acylated acyl acceptor.

8. The method of claim 7, wherein the separation is by use of an organic and an aqueous phase.

9. The method of any of claims 6 to 8, wherein the activity is determined by detecting radioactivity.

10. The method of any of claims 6 to 8, wherein the activity is determined by detecting fluorescence.

11. The method of any of claims 6 to 10, wherein determining the acyl transferring activity of MEST is by determining the amount of acyl glycerol 3-phosphate.

12. The method of any of claims 6 to 11, wherein the method is a homogenous assay.

13. The method of any of claims 6 to 12, wherein the acyl transferring activity of MEST is decreased relative to a control, thereby identifying the substance as a MEST inhibitor.

14. The method of claims 6 and 13, wherein the method is used for screening for a medicament for preventing and/or treating obesity and/or diabetes.

15. Use of a test system according to any of claims 1 to 5 for the identification of a MEST ligand, particularly a MEST inhibitor.


SEQUENCE LISTING

<110>  Sanofi-Aventis Deutschland GmbH

<120>  Test system for measuring MEST activity as well as methods and
       uses involving the same

<130>  DE2009/100

<160>  3

<170>  PatentIn version 3.5

<210>  1
<211>  335
<212>  PRT
<213>  Homo sapiens

<400>  1

<210>  2
<211>  31
<212>  DNA
<213>  Artificial Sequence

<220>
<223>  Primer

<400>  2
gagagaattc gatggtgcgc cgagatcgct t                                    31

<210>  3
<211>  32
<212>  DNA
<213>  Artificial Sequence

<220>
<223>  Primer

<400>  3
gagagcggcc gctcagaagg agttgatgaa gc                                   32